(19) Europäisches Patentamt
European Patent Office
Office européen des brevets

(11) EP 1 591 436 A2

(12) # EUROPÄISCHE PATENTANMELDUNG

(43) Veröffentlichungstag:
**02.11.2005 Patentblatt 2005/44**

(51) Int Cl.[7]: **C07C 51/58**, C07C 53/48, C07C 53/40, C01B 17/45

(21) Anmeldenummer: **05016647.9**

(22) Anmeldetag: **24.11.1999**

(84) Benannte Vertragsstaaten:
**AT BE CH CY DE DK ES FI FR GB GR IE IT LI LU MC NL PT SE**

(30) Priorität: **30.11.1998 DE 19855252**
**13.07.1999 DE 19932554**
**04.09.1999 DE 19942374**

(62) Dokumentnummer(n) der früheren Anmeldung(en) nach Art. 76 EPÜ:
**99959316.3 / 1 135 356**

(71) Anmelder: **Solvay Fluor GmbH**
**30173 Hannover (DE)**

(72) Erfinder:
- **Braun, Max**
  **3090 Wedemark (DE)**
- **Rieland, Matthias**
  **30559 Hannover (DE)**
- **Janssens, Francine**
  **1800 Vilvoorde (BE)**
- **Eichholz, Kerstin**
  **30855 Langenhagen (DE)**
- **Palsherm, Stefan**
  **30890 Barsinghausen (DE)**

(74) Vertreter: **Fischer, Reiner et al**
**Solvay Fluor GmbH,**
**Hans-Böckler-Allee 20**
**30173 Hannover (DE)**

Bemerkungen:
Diese Anmeldung ist am 01-08-2005 als Teilanmeldung zu der unter INID-Kode 62 erwähnten Anmeldung eingereicht worden.

(54) **Verfahren zur Herstellung von Säurefluoriden aus Säurechloriden**

(57) Säurefluoride, beispielsweise Carbonsäurefluoride und Sulfurylfluorid, können hergestellt werden, indem die entsprechenden Säurechloride mit Fluorwasserstoff-Addukten von Ammoniumfluorid oder Aminhydrofluoriden (diese wirken als Katalysator oder als Fluorierungsmittel) umgesetzt werden. Verbrauchte HF-Addukte können mit HF regeneriert werden.

EP 1 591 436 A2

## Beschreibung

Die Erfindung bezieht sich auf ein Verfahren zur Herstellung von Säurefluoriden aus Säurechloriden unter Kontaktieren mit Fluorwasserstoff-Addukten von Stickstoffbasen-Hydrofluoriden.

Säurefluoride, beispielsweise Carbonsäurefluoride und Sulfurylfluorid, sind interessante Produkte zur Verwendung per se oder als Zwischenprodukt. Sulfurylfluorid beispielsweise kann als Entwesungsmittel ("Fumigant") für Schädlinge, z. B. in verbautem Holz, Kirchen, Museen, Silos und Gebäuden und gegen verfärbende Enzyme, Pilze oder Krankheitserreger in nicht verbautem, z. B. frisch gefälltem Holz eingesetzt werden. Carbonsäurefluoride sind wertvolle Zwischenprodukte in der chemischen Synthese. Sulfurylchloridfluorid kann zu Sulfurylfluorid umgesetzt werden.

Die deutsche Offenlegungsschrift DE-OS 28 23 969 offenbart die Herstellung von Fluorcarbonyl-Verbindungen aus Chlorcarbonyl-Verbindungen, beispielsweise von Chlordifluoracetylfluorid aus Chlordifluoracetylchlorid und HF-Addukten von Hydrofluoriden organischer Stickstoffbasen als Fluorierungsmittel. Verwendet man HF-Addukte, wird Amin zur HF-Bindung zugesetzt oder es werden Lösungsmittel mit ausreichender Basizität zur HF-Bindung verwendet.

R. Franz offenbart in J. Fluorine Chem. 15 (1980), Seiten 423 bis 434, dass die an sich schon bekannten Trishydrofluoride tertiärer Amine als Fluorierungsmittel beim nucleophilen Austausch von Chlor- und Bromatomen brauchbar sind. Beispielsweise lassen sich Difluorphosgen, Oxalylfluorid, Cyanurfluorid und Schwefeltetrafluorid herstellen.

Aufgabe der vorliegenden Erfindung war es, ein verbessertes Fluorierungsverfahren für die Herstellung von Säurefluoriden aus Säurechloriden anzugeben. Diese Aufgabe wird durch das Verfahren der vorliegenden Erfindung gelöst.

Erfindungsgemäß stellt man Sulfurylfluorid, Sulfurylchloridfluorid oder Carbonsäurefluoride der allgemeinen Formel (I) her,

$$RC(O)F \qquad I$$

worin R bedeutet: C1-C7-Alkyl; durch mindestens 1 Chloratom und/oder durch mindestens 1 Fluoratom substituiertes C1-C7-Alkyl, durch Kontaktieren von Säurechloriden und Fluorwasserstoff-Addukten von Ammoniumhydrofluorid oder Hydrofluoriden organischer Stickstoffbasen ohne Zusatz einer HF-bindenden Base oder eines HF-bindenden Lösemittels, wobei

das Fluorwasserstoff-Addukt als Fluorierungsreagenz dient und nicht über die Stufe des Ammoniumhydrofluorids oder des Hydrofluorids der organischen Stickstoffbase hinaus dehydrofluoriert wird, oder

wobei man mindestens 1 Mol HF pro Mol auszutauschenden Chloratom einsetzt und die Fluorwasserstoff-Addukte als Katalysator der Umsetzung der Säurechloride mit HF dienen, oder

wobei die Fluonwasserstoff-Addukte regeneriert werden, um enthaltene HCl zu entfernen.

Dabei arbeitet man, anders als im Stand der Technik, so, dass keine Base zugesetzt wird, die HF-bindend wirkt. Es wird auch kein HF-bindendes Lösungsmittel verwendet. Allenfalls, wenn man gemäß einer noch zu beschreibenden Ausführungsform eine Säure zusetzt, wie z. B. Trifluoressigsäure, kann man diese Säure oder einen Teil dieser Säure durch Base neutralisieren, indem man vor, während oder nach der Säurezugabe eine gewünschte Basenmenge zusetzt. HF wird aber nicht gebunden.

Zur Kontaktierung sind - neben HF-Addukten von Ammoniumfluorid - die in der deutschen Offenlegungsschrift DE-OS 28 23 969 genannten HF-Addukte von Hydrofluoriden organischer Stickstoffbasen brauchbar. Sie können durch die Formel $B\cdot(HF)_x$ ausgedrückt werden, darin stellt B eine organische Stickstoffbase dar und x bedeutet eine ganze oder gebrochene Zahl von >1 bis 4, vorzugsweise 2 - 3.

Als organische Stickstoffbasen B können alle möglichen primären, sekundären und/oder tertiären Amine einschließlich N-Heterozyklen verwendet werden. Wenn man als Formel für diese Amine angibt:

$$R_1R_2R_3N$$

so können darin bedeuten:

$R_1$ einen Alkylrest, vorzugsweise mit 1 bis 10, insbesondere mit 1 bis 6 C-Atomen,
einen Cycloalkylrest, vorzugsweise mit 5 bis 7 C-Atomen,
einen Aralkylrest, vorzugsweise mit 6 bis 10 C-Atomen
oder
einen Arylrest, vorzugsweise ebenfalls mit 6 bis 10 C-Atomen;

$R_2$ und $R_3$ Wasserstoff,
Alkyl-, Cycloalkyl-, Aralkyl- und Arylreste der gleichen Art wie bei $R_1$ angegeben.

Die Reste $R_2$ und $R_3$ können gleich oder verschieden sein. Zwei der Reste $R_1$ und $R_2$ oder $R_3$ können auch zu einem cycloaliphatischen Ring, welcher gegebenenfalls noch durch andere Heteroatome wie durch Sauerstoffatome unterbrochen sein kann, geschlossen sein. Ebenfalls ist es möglich, dass die drei Reste $R_1$, $R_2$ und $R_3$ Bestandteil eines heterocyclischen Ringes sind, wodurch dann entsprechende N-Heterocyclen resultieren. Bevorzugte organische Stickstoffbasen B sind primäre, sekundäre und/oder tertiäre Amine mit insgesamt bis zu 12 C-Atomen, wobei die sekundären und/oder tertiären aliphatischen Amine besonders bevorzugt sind.

Konkrete Beispiele für die Basen B sind:

N-Butylamin, N-Decylamin, Diäthylamin, Di-n-octylamin, Trimethylamin, Triäthylamin, Tri-n-propylamin, Isopropyldiäthylamin, Tri-n-butylamin, Cyclohexylamin, N-Methylanilin, N,N-Dimethylanilin, Pyrrolidin, Piperidin, N-Methylpiperidin, Morpholin, Pyridin, Chinolin etc.

Die HF-Addukte der Hydrofluoride der Stickstoffbasen B sind leicht aus den Basen B und Fluorwasserstoff erhältlich; sie sind niedrigschmelzende oder bei Raumtemperatur flüssige Substanzen mit beträchtlicher thermischer Belastbarkeit. Die Trishydrofluoride sind sogar unzersetzt vakuumdestillierbar.

Bevorzugt setzt man HF-Addukte von primären, sekundären oder tertiären Aminhydrofluoriden mit bis zu 15 C-Atomen ein, insbesondere von sekundären und tertiären Aminhydrofluoriden. Besonders gut geeignet sind HF-Addukte von Tri-n-propylaminhydrofluorid, Tri-iso-propylaminhydrofluorid, Tri-n-butylaminhydrofluorid, Pyridinhydrofluorid, Piperidinhydrofluorid oder N,N-Dimethylaminhydrofluorid.

Man führt die Umsetzung in flüssiger Phase durch.

Das erfindungsgemäße Verfahren kann batchweise oder kontinuierlich durchgeführt werden. Bei kontinuierlicher Fahrweise kann man so vorgehen, dass neben dem zu fluorierenden Säurechlorid auch HF, frisches HF-Addukt oder beides eingespeist wird. Entsprechend wird Reaktionsmischung abgetrennt bzw. gasförmige Reaktionsprodukte abdestilliert oder gasförmig abgeführt.

Die Erfinder haben festgestellt, dass sich im Laufe der Zeit HCl in der Reaktionsmischung anreichert. Wenn nicht intermittierend oder kontinuierlich HF eingespeist wird, verarmt die Reaktionsmischung bzw. das HF-Addukt an HF. Es wurde gefunden, dass das HF-Addukt durch Behandlung mit HF, gegebenenfalls bei erhöhter Temperatur (80 bis 120 °C) und erhöhtem Druck (z. B. autogener Druck im Autoklaven) regeneriert werden kann. Dabei zeigte es sich, dass es nicht notwendig ist, vorhandenes HCl vollständig auszutreiben. Ein Restgehalt von z. B. weniger als 5 Gew.-%, vorzugsweise weniger als 2 Gew.-% HCl ist akzeptabel.

Gemäß einer Ausführungsform der Erfindung stellt man Sulfurylfluorid als Säurefluorid aus Sulfurylchlorid oder Schwefeldioxid und Chlor (dann unter Druck zwecks Verflüssigung) her. Hier setzt man - zur Freisetzung von HCl - eine Säure zu, beispielsweise eine halogenierte Carbonsäure wie Trifluoressigsäure. Es kann, zwecks (Teil)Neutralisierung dieser zugesetzten Säure, eine Stickstoffbase zugesetzt werden. Dabei entspricht zweckmäßig diese Base der im Hydrofluorid enthaltenen Base.

Sulfurylfluorid kann auch aus Sulfurylchloridfluorid hergestellt werden. Es ist somit eine zweistufige Herstellung von Sulfurylflüorid möglich. Die erste Stufe umfasst die Herstellung von Sulfurylchloridfluorid aus Sulfurylchlorid. Das Verhältnis von Amin (bzw. $NH_3$) zu HF in der Reaktionsmischung ist bei dieser Stufe nicht begrenzt; die Durchführung gelingt auch mit sehr hohem HF-Gehalt, z. B. bei einem Verhältnis von Amin:HF von 1:3 bis hin zu 1:10 oder auch darüber. Die zweite Stufe, die Fluorierung von Sulfurylchloridfluorid zu Sulfurylfluorid, verlangt ein Verhältnis von Amin zu HF, das größer ist als 1:3; beispielsweise liegt es zwischen 1:2 und 1:3. Diese Maßgabe bezüglich des maximalen Gehalts an HF in der Reaktionsmischung gilt auch bei der einstufigen Herstellung von $SO_2F_2$ aus $SO_2Cl_2$, wenn eine gute Ausbeute an $SO_2F_2$ erzielt werden soll. Es wird angenommen, dass bei einem Verhältnis von Amin: HF unterhalb von 1:3 sich die Reaktivität (Nucleophilie) des F--Anions ändert.

Die zweistufige Herstellung von Sulfurylfluorid ermöglicht es, eine besondere Verfahrensvariante anzuwenden. Es wurde nämlich festgestellt, dass während der Fluorierung von $SO_2Cl_2$ zu $SO_2ClF$ gleichzeitig die Regenerierung des HF-Adduktes (wie oben erwähnt) möglich ist. Man bringt $SO_2Cl_2$, einen Überschuss HF und zu regenerierendes HF-Addukt in einen Reaktor ein. Unter erhöhtem Druck (z. B. autogener Druck im Autoklaven) wird simultan $SO_2ClF$ und regeneriertes HF-Addukt erzeugt. Gasförmiges HCl, das sich bildet, wird abgetrennt (z. B. durch Ablassen des Überdrucks und Durchleiten von Inertgas wie $N_2$). Dann wird HF abgedampft, um den HF-Gehalt auf die obenbeschriebene Limitierung zu bringen (Amin:HF >1:3). Dann kann die zweite Stufe zwecks Herstellung von $SO_2F_2$ durchgeführt werden. Werden die erste und zweite Stufe im gleichen Reaktor durchgeführt, ist es so möglich, in die zweite Stufe stets mit refluoriertem HF-Addukt zu gehen.

Gemäß einer anderen Ausführungsform stellt man Carbonsäurefluoride der Formel I

RC(O)F

her, worin R für C1 - C7-Alkyl; durch mindestens 1 Chlor-Atom und/oder durch mindestens 1 Fluor-Atom substituiertes C1 - C7-Alkyl steht. Besonders bevorzugt steht R für C1 - C3-Alkyl; oder für durch mindestens 1 Chlor-Atom und/oder durch mindestens 1 Fluor-Atom substituiertes C1 - C3-Alkyl. Ganz besonders steht R für $CH_3$, $C_2H_5$, $CF_3$, $CF_2Cl$, $CFCl_2$, $CCl_3$, $CHF_2$, $C_2F_5$ oder $C_3F_7$.

R kann aber auch für aromatische Reste wie Phenyl oder Tolyl stehen.

Den Chlor-Fluor-Austausch gemäß der vorliegenden Erfindung führt man vorzugsweise bei einer Temperatur von Umgebungstemperatur (etwa 20 °C) bis 150 °C durch. Bevorzugt liegt das Molverhältnis des HF-Adduktes des Hydrofluorides, bezogen auf die darin enthaltene Base, zu Säurechlorid im Bereich von 1:0,01 bis 1:1 (1 Mol $R_3N{\cdot}2{,}6$ HF und 1 Mol Säurechlorid haben dann ein Verhältnis von 1:1), wenn pro Säurechlorid-Addukt ein Cl-Atom ausgetauscht werden soll. Bei mehreren Chlor-Atomen, die pro Molekül ausgetauscht werden sollen, ist der Einsatz des Hydrofluorids zweckmäßig doppelt, dreifach etc. so hoch. Bei kontinuierlicher Verfahrensweise kann das Verhältnis im Bereich von 1:0,01 bis 1:100 liegen.

Bei der Umsetzung wird bei der Herstellung von Carbonsäurefluoriden spontan Chlorwasserstoff freigesetzt. Dieser Chlorwasserstoff kann aus dem Reaktor abgelassen werden (beispielsweise durch ein entsprechend eingestelltes Überdruckventil). Bei der Herstellung von $SO_2F_2$ ist zur HCl-Freisetzung die Zugabe von Säuren wie Trifluoressigsäure erforderlich.

Es wurde bei Versuchen festgestellt, dass oftmals ein Zusatz von z. B. Trifluoressigsäure gleich bei Beginn der Fluorierungsreaktion insofern vorteilhaft ist, als das Ausfallen von Feststoffen (die sich später wieder auflösen) verhindert oder reduziert wird. Beispielsweise reichen schon 10 Mol-% der Säure, bezogen auf das als 100 Mol-% berechnete Onium-HF-Addukt, aus.

Es wurde auch festgestellt, dass Ammoniumsalze mit drei C1- oder C2-Alkylresten gebildetes HCl sehr leicht freisetzen; sie neigen allerdings zur Bildung von Feststoffen, so dass der Zusatz von z. B. Trifluoressigsäure, wie vorstehend beschrieben, vorteilhaft ist. Oniumsalze mit drei C3- oder höherkettigen Alkylresten werden zwar nicht fest; aber aus ihnen wird HCl nicht so leicht freigesetzt wie aus den kürzerkettig substituierten Oniumsalzen. Hier ist der Säurezusatz vorteilhaft, weil dadurch verstärkt HCl ausgetrieben wird.

Gemäß einer Ausführungsform fungiert das Hydrofluorid-Addukt als Fluorierungsmittel. Es wird dann in einer solchen Menge eingesetzt, dass es nicht so weit dehydrofluoriert wird, dass die Stufe des Oniummonohydrofluorids überschritten wird. Wenn beispielsweise ein Addukt der Formel $R_3N{\cdot}2{,}6$ HF eingesetzt wird, soll nur soviel HF verbraucht werden, dass $R_3N{\cdot}zHF$ mit z=1 oder z>1 im Reaktionsgemisch verbleibt. Das entsprechende Hydrochlorid soll nicht entstehen, wenn man möglichst lange Zeit eine Regenerierung unter Druck mit HF vermeiden will. Es genügt hier, lediglich HF zuzusetzen.

Wenn beispielsweise bei einer Verbindung ein Chloratom gegen ein Fluor-atom ausgetauscht werden soll, setzt man pro 1,6 Mol der Ausgangsverbindung mindestens 1 Mol des Adduktes $R_3N{\cdot}2{,}6$ HF ein. Setzt man andere Edukte (z. B. $SO_2Cl_2$) oder Oniumsalze mit anderem HF-Gehalt ein, so ist die Stöchiometrie entsprechend anzupassen.

Gemäß einer anderen Ausführungsform fungiert das Hydrofluorid-Addukt als Katalysator. Als Fluorierungsmittel wird dann noch HF in die Reaktion eingebracht. Die Menge an HF beträgt dann vorteilhaft mindestens 1 Mol HF pro auszutauschendem Chloratom. Das Verhältnis der Summe von freiem und im Addukt gebundenem HF zum auszutauschenden Chloratom kann z. B. im Bereich von etwa 1:1 bis hin zu 1:3 liegen, wenn $SO_2F_2$ aus $SO_2Cl_2$ oder $SO_2FCl$ hergestellt werden soll. Es kann bei der Herstellung von Carbonsäurefluoriden oder $SO_2FCl$ auch noch höher liegen, wenn HF als Lösemittel fungieren soll. Man kann auch weniger als 1 Mol HF pro auszutauschendem Chloratom einsetzen; dann wird das HF aus dem an sich katalytisch wirkenden HF-Addukt verbraucht, bzw. die Ausbeute sinkt.

Gegenüber dem bekannten Verfahren fungiert das Hydrofluorid von Ammoniak bzw. der organischen Stickstoffbase hier nicht als Fluorierungsmittel und Reaktionspartner, sondern als Katalysator. Deswegen wird erstmals eine kontinuierliche Verfahrensweise möglich. Der freigesetzte Chlorwasserstoff kann gewünschtenfalls kontinuierlich aus dem Reaktionssystem abgelassen werden oder bei der Regenerierung entfernt werden. Es ist nicht notwendig, ein Lösungsmittel zu verwenden.

Somit weist das Verfahren gemäß der Erfindung den Vorteil auf, dass die Aufarbeitung sehr viel einfacher ist: es fällt kein Aminhydrochlorid als Abfallprodukt an; man muss nicht von einem Lösemittel abtrennen.

Ein weiterer Gegenstand der Erfindung ist eine Zusammensetzung, die fluorierend wirkt. Sie ist erhältlich durch Vermischen von HF-Addukten der Formel $B^1{\cdot}mHF$ und einer Säure. Diese wirkt derart, dass HCl aus der Reaktionsmischung bei der $SO_2F_2$-Herstellung freigesetzt wird. Gegebenenfalls kann noch Base $B^1$ zugesetzt sein, in einer Menge, die geringer ist als die zur Neutralisation der Säure notwendige Menge. $B^1$ bedeutet $NH_3$ oder die Base B, wie oben definiert, m bedeutet $1 < m < 4$. Bevorzugte Säure ist eine halogenierte Carbonsäure wie Trifluoressigsäure. Das HF-Addukt von $B^1$ kann auch in situ erzeugt werden. Gewünschtenfalls kann man diese Säure oder einen Teil der Säure in Form des Salzes mit $B^1$ einbringen. Die bevorzugte Zusammensetzung hat die "Formel" $B^1{\cdot}(0.1\text{-}1{,}0)$TFA·

(1,0-3,0)HF. TFA ist Trifluoressigsäure. $B^1$ ist bevorzugt B. Bevorzugtes B ist weiter oben angegeben.

Die folgenden Beispiele sollen die Erfindung weiter erläutern, ohne sie in ihrem Umfang einzuschränken. Die Beispiele 1 bis 6 erläutern die Verwendung von HF-Addukten als Fluorierungsmittel (vermutlich autokatalytisch), die Beispiele 7 bis 8 die Verwendung als Katalysator.

**Beispiele**

**Beispiel 1:**

Herstellung von Chlordifluoracetylfluorid (CDFAF) aus Chlordifluoracetylchlorid (CDFAC) unter Verwendung von Tributylamin·2,6 HF

$$ClF_2C\text{-}COCl+(CH_3\text{-}CH_2\text{-}CH_2\text{-}CH_2)_3N\cdot 2{,}6\ HF \longrightarrow ClF_2C\text{-}COF+HCl$$

| Ansatz: | |
|---|---|
| 0,90 Mol Chlordifluoracetylchlorid (CDFAC) | 134,0 g |
| 0,56 Mol Tributylamin·2,6 HF | 133,5 g |
| 0,50 Mol Chlordifluoressigsäure | 74,5 g |

Aufbau u. Durchführung:

In einem 250 ml Dreihalskolben mit Rückflusskühler, Thermofühler und Tropftrichter wurde das Tributylamin·2,6 HF vorgelegt. Der Rückflusskühler wurde über einen Kryomaten mit -20 °C kalter Sole gespeist. Hinter dem Rückflusskühler befand sich ein Blasenzähler, der das Entweichen von gasförmigen Produkten anzeigte. Um das Reaktionsprodukt aufzufangen, war nach dem Rückflusskühler ein 300 ml Stahlzylinder mit Tauchrohr geschaltet, der in einem Dewar mit $CO_2$ /Methanol auf -78 °C temperiert wurde.

Bei Raumtemperatur wurde nun langsam und unter starkem Rühren CDFAC in die Lösung getropft. Die Reaktion war leicht exotherm, wobei die Temperatur nicht über 31 °C stieg. Sofort nach Beginn des Zutropfens war eine Gasentwicklung zu beobachten. Nach 15 Minuten erfolgte die erste Probenahme nach dem Kühler. Während der Reaktion wurden mehrere Proben genommen und nach etwa zwei Dritteln der Stöchiometrie wurde im Abgasstrom auch HCl detektiert. Nach einer Stunde wurde der Kryomat auf -30 °C gestellt, weil zuviel CDFAC entwich. Nach vollständiger Zugabe des CDFAC's wurde noch so lange nachgerührt, bis die Gasentwicklung (Blasenzähler) beendet war. Anschließend wurde nochmals zum vollständigen Austreiben der Reaktionsprodukte im Wasserbad auf 50 °C temperiert, bis am Blasenzähler kein Entweichen von Reaktionsprodukten mehr erkennbar war. Nun wurde noch die CDFA zugegeben, um HCl vollständg auszutreiben.

Auswertung:

Die Gasproben zeigten zu Beginn der Reaktion einen Gehalt von 91 % Chlordifluoracetylfluorid und 7 % mitgeschlepptes Chlordifluoracetylchlorid (GC-Flächenprozent) und zunächst noch kein HCl. Nach Zutropfen von insgesamt 0,6 Mol CDFAC wurde nun auch die entstehende HCl aus der Reaktionslösung freigesetzt und mit GC-MS bestätigt. Im Stahlzylinder befand sich eine Mischung aus 91,7 % CDFAF und 6,9 % aus der Reaktion mitgeschlepptem CDFAC neben 1,4 % HCl (aufgrund der für eine HCl-Kondensation zu niedrigen Temperatur wurde die entstandene HCl nicht im Stahlzylinder einkondensiert). Die isolierte Ausbeute an CDFAF und CDFAC betrug 89,5 % der Theorie.

**Beispiele zur Herstellung von $SO_2F_2$ aus $SO_2Cl_2$ unter Verwendung von Tributylamin·2,6 HF und Tributylamin·TFA·2,6 HF**

$$SO_2Cl_2 + 2\ (CH_3\text{-}CH_2\text{-}CH_2\text{-}CH_2)_3\ N\cdot 2{,}6\ HF + TFA \rightarrow SO_2F_2 + 2\ HCl$$

**Beispiel 2:**

nachträgliche Zugabe von TFA zur Freisetzung der HCl

| Anzsatz: | |
|---|---|
| 0,30 Mol Sulfurylchlorid ($SO_2Cl_2$) | 40,5 g |
| 0,375 Mol Tributylamin·2,6 HF | 71,2 g |
| 0,375 Mol Trifluoressigsäure | 42,8 g |

Aufbau und Durchführung aus diesem Beispiel gilt auch für alle nachfolqenden Beispiele:

In einem 250 ml Dreihalskolben mit Rückflusskühler, Thermofühler und Topftrichter wurde der Tributylamin-Komplex vorgelegt. Der Rückflusskühler wurde über einen Kryomaten mit -30 °C kalter Sole gespeist. Ein nach dem Rückflusskühler installierter Blasenzähler zeigte das Verlassen von Produkten über den Rückflusskühler an. Um das Reaktionsprodukt aufzufangen, war nach dem Kühler ein Stahlzylinder (mit ca. 300 ml Volumen) mit Tauchrohr und Gasausgang geschaltet, der in einem Dewar mit $CO_2$ /Methanol auf -78 °C temperiert wurde. Bei Raumtemperatur wurde langsam und unter starkem Rühren $SO_2Cl_2$ in die Lösung getropft. Die Reaktion war leicht exotherm. Kurze Zeit nach Beginn des Zutropfens war eine Gasentwicklung zu beobachten, die GC-Analyse des Gases nach dem Rückflusskühler zeigte 85,6 % $SO_2F_2$ neben 14,3 % $SO_2FCl$ (keine HCl) an. Zu Beginn der Reaktion färbte sich die Lösung im Kolben etwas dunkler, wurde jedoch nach einiger Zeit wieder hell, fast farblos.

Da bisher noch keine HCl im Abgas detektiert wurde (notwendig im Einsatz für katalytische Fluorierung) wurde nach Beendigung der Gasentwicklung (Beobachtung Blasenzähler) aus obiger Reaktion vorsichtig Trifluoressigsäure (TFA) zugetropft. Sofort wurde HCl und auch noch weitere Mengen $SO_2F_2$ aus der Reaktionslösung freigesetzt. Um das Austreiben von Reaktionsprodukten und die Reaktion zu vervollständigen, wurde die Reaktionslösung auf 50 °C temperiert bis am Blasenzähler keine Gasentwicklung mehr zu beobachten war. Die isolierte Ausbeute im Zylinder betrug wegen der unzureichenden Kühlung nur 48 %.

**Beispiel 3:**

Versuch mit einem TFA/$NBu_3$-Verhältnis von 1:1/Sofortiger Einsatz von $Bu_3N$/HF/TFA als Katalysatormischung

| Ansatz: | |
|---|---|
| 0,23 Mol $SO_2Cl_2$ | 31,7 g |
| 0,20 Mol Tributylamin·2,6 HF | 47,5 g |
| 0,123 Mol Tributylamin | 22,8 g |
| 0,323 Mol Trifluoressigsäure | 36,8 g |

Aufbau:

Wie bei Beispiel 2.

Herstellung der fluorierenden Zusammensetzung:

Das HF-Addukt des Tributylamins und das Tributylamin wurden miteinander vermischt. Dann wurde langsam die Trifluoressigsäure zugetropft.

Durchführung der Fluorierung:

Das $SO_2Cl_2$ wurde langsam zugetropft. Im Abgas konnte sofort HCl sowie $SO_2F_2$ und $SO_2FCl$ nachgewiesen werden.

**Beispiel 4:**

Versuch mit $NBu_3$/TFA = 1:0,1

| Ansatz: | |
|---|---|
| 0,35 Mol $SO_2Cl_2$ | 47,2 g |
| 0,30 Mol Tributylamin·2,6 HF | 71,2 g |
| 0,03 Mol Tributylamin | 5,6 g |
| 0,03 Mol Trifluoressigsäure | 3,4 g |

Aufbau und Durchführung:

Wie bei Beispiel 3.

Das Abgas hatte beim ersten Zutropfen von $SO_2Cl_2$ eine Zusammensetzung von 73,4 % $SO_2F_2$ und 25,1 % $SO_2FCl$ und anfangs noch keine HCl. Später veränderte sich die Zusammensetzung etwas zu Gunsten des Sulfurylfluorides auf 97,2 % $SO_2F_2$ und nur noch 2,0 % $SO_2FCl$ und 0,3 % HCl. Eine Massenbilanz wurde nicht erstellt.

**Beispiel 5:**

Versuch mit $NBu_3$/TFA = 1 : 0,23

| Ansatz: | |
|---|---|
| 0,35 Mol $SO_2Cl_2$ | 47,2 g |
| 0,30 Mol Tributylamin 2,6 HF | 71,2 g |
| 0,09 Mol Tributylamin | 16,7 g |
| 0,09 Mol Trifluoressigsäure | 10,34 g |

Aufbau und Durchführung:

Wie bei Beispiel 3. In diesem Experiment wurde ein geeigneter Stahlzylinder mit flüssigem Stickstoff gekühlt, um eine Massenbilanz zu erhalten.

Das Abgas hatte zu Beginn des $SO_2Cl_2$-Zutropfens eine Zusammensetzung von 85,1 % $SO_2F_2$ und 14,8 % $SO_2FCl$ und 0,6 % HCl. Die im Stahlzylinder einkondensierte Mischung bestand aus
82 % $SO_2F_2$ und 18 % $SO_2FCl$ und Spuren von HCl und $SO_2$. Das entspricht einer theoretischen Ausbeute von 90,3 % bezogen auf isolierte Mengen an $SO_2F_2$ und $SO_2FCl$.

**Beispiel 6:**

Herstellung von Trifluoracetylfluorid (TFAF) aus Trifluoracetylchlorid (TFAC) mit Tributylamin·2,6 HF als Katalysator

| Ansatz: | |
|---|---|
| 104,2 g Tributylamin·2,6 HF | 0,44 Mol |
| 151,0 g Trifluoracetylchlorid | 1,14 Mol |

Aufbau und Durchführung:

In einem 250 ml Mehrhalskolben mit Rührfisch, Thermofühler, und aufgesetztem Kühler (-50 °C) und Blasenzähler am Ausgang wurde das Tributylamin·2,6 HF vorgelegt und unter Rühren bei Raumtemperatur TFAC eingeleitet.

Die Reaktion wurde leicht exotherm (T max. 32 °C). Es erfolgte eine spontane Gasentwicklung. Während der Reaktion verfärbte sich die Lösung von hell- nach mittelbraun. Nach Ende des Versuches zeigte sich im Sumpf eine Korrosion am Glas.

Eine Abgasprobe nach dem Blasenzähler enthielt 88,3 % TFAF neben TFAC und HCl. Eine Massenbilanz wurde nicht erstellt.

**Beispiele 7 und 8:**

Versuche zur Verwendung von $NBu_3 \cdot 2{,}6$ HF als Fluorierungskatalysator

**Beispiel 7:**

Kontinuierliches Verfahren unter Verwendung von Tributylamin·2,6 HF (ohne TFA) als Katalysator

| Ansatz: | |
|---|---|
| 0,74 Mol Sulfurylchlorid ($SO_2Cl_2$) | 100,00 g |
| 0,40 Mol Tributylamin·2,6 HF | 94,9 g |
| 0,46 Mol 1,1,1,3,3-Pentafluorbutan (S 365mfc) | 68,3 g |
| 1,48 Mol HF | 29,6 g |

Aufbau und Durchführung (gilt für das nachfolgende Beispiel):

In einer 250 ml PFA Waschflasche wurden 365 und Sulfurylchlorid vorgelegt und mit Eis gekühlt. Dann wurde vorsichtig unter Rühren HF eingeleitet. In einem 250 ml Dreihalskolben mit Rückflusskühler, Thermofühler und Topftrichter wurde der Tributylaminkomplex vorgelegt. Der Rückflusskühler wurde über einen Kryomaten mit -30 °C kalter Sole gespeist. Bei Raumtemperatur wurde langsam und unter starkem Rühren die Mischung aus HF/S365/$SO_2Cl_2$ in die ölige, hellbraune Lösung getropft. Die Reaktion ist exotherm ($\Delta T$ = 22 °K), die Innentemperatur stieg bis auf die Siedetemperatur des 365mfc an (41 °C). Das 365mfc wurde durch den Kondensator im Reaktionskolben zurückgehalten. Kurze Zeit nach Beginn des Zutropfens ist eine Gasentwicklung zu beobachten; neben HCl wurde $SO_2F_2$ und hauptsächlich $SO_2FCl$ freigesetzt. Eine Massenbilanz wurde nicht erstellt.

**Beispiel 8:**

Kontinuierliches Verfahren unter Verwendung von Tributylamin·2,6 HF mit TFA als Katalysator

| Ansatz: | |
|---|---|
| 0,80 Mol Sulfurylchlorid ($SO_2Cl_2$) | 107,98g |
| 0,20 Mol Tributylamin·2,6 HF | 47,74g |
| 1,60 Mol HF | 32,0 g |
| 0,77 Mol S 365mfc | 113,3 g |
| 0,02 Mol Tributylamin | 3,7 g |
| 0,02 Mol Trifluoressigsäure | 2,3 g |

Aufbau und Durchführung:

Siehe oben.

Kurze Zeit nach Beginn des Zutropfens war eine Gasentwicklung zu beobachten; neben HCl wurde $SO_2F_2$ und hauptsächlich $SO_2FCl$ freigesetzt. Das 365mfc wurde durch den Kondensator weitgehendst im Reaktionskolben zurückgehalten. Die HCl-Entwicklung erfolgte etwas früher als im vorherigen Versuch. Eine Massenbilanz wurde nicht erstellt.

**Beispiel 9:**

Verwendung von $NEt_3$·2,6 HF als Fluorierungskatalysator zur Herstellung von $SO_2F_2$

$$NEt_3 \cdot 2{,}6\ HF + SO_2Cl_2 \rightarrow SO_2F_2$$

Ansatz:

| Stoff | Molmasse | Masse in g | Mol |
|---|---|---|---|
| Triethylamin·2,6 HF | 153,22 | 58,2 | 0,38 |
| Sulfurylchlorid | 134,97 | 41,6 | 0,31 |

Durchführung:

In einem Dreihalskolben mit Glaskühler (~0 °C) und abschließendem Blasenzähler wurde Triethylamin·2,6 HF vorgelegt und unter Rühren $SO_2Cl_2$ bei Raumtemperatur zugetropft. Zu Beginn des $SO_2Cl_2$-Zutropfens hatte das den Kühler verlassende Abgas eine Zusammensetzung von 63 % $SO_2F_2$ neben 37 % $SO_2FCl$. Gegen Ende der Reaktion fiel der $SO_2F_2$-Gehalt bis auf 56 % $SO_2F_2$ ab. Eine Massenbilanz wurde nicht erstellt.

**Beispiel 10:**

Herstellung von $SO_2FCl$ aus $SO_2Cl_2$ unter Verwendung von Tributylamin·4,5 HF

$$SO_2Cl_2 + (CH_3\text{-}CH_2\text{-}CH_2\text{-}CH_2)_3N\cdot 4{,}5\ HF \rightarrow SO_2FCl + HCl$$

| Ansatz: | |
|---|---|
| 0,24 Mol Sulfurylchlorid ($SO_2Cl_2$)<br>0,24 Mol Tributylamin·4,5 HF | 47,2 g<br>68,4 g |

Aufbau und Durchführung:

In einem 250 ml Dreihalskolben mit Rückflusskühler mit Blasenzähler, Thermofühler und Tropftrichter wurde das Tributylamin vorgelegt. Der Rückflusskühler wurde über einen Kryomaten mit ca. 0 °C kalter Sole gespeist. Bei Raumtemperatur wurde langsam und unter starkem Rühren $SO_2Cl_2$ in die ölige, hellbraune Lösung zugetropft. Die Reaktion war leicht exotherm (ΔT = 10 °K). Kurze Zeit nach Beginn des Zutropfens war eine Gasentwicklung zu beobachten. Als Reaktionskontrolle wurden während des Zutropfens GC-Proben aus dem Abgasstrom nach dem Blasenzähler genommen. Zu Beginn der Zugabe von $SO_2Cl_2$ betrug die Abgaszusammensetzung 55,2 % HCl, neben 42,07 % $SO_2FCl$; auch $SO_2$ wurde detektiert. Bei Beendigung des Zutropfens von $SO_2Cl_2$ war die Abgaszusammensetzung nahezu identisch. Eine Massenbilanz wurde nicht erstellt.

**Beispiel 11:**

Herstellung von Acetylfluorid aus Acetylchlorid mit Triethylamin·2,6 HF als Katalysator

| Ansatz: | |
|---|---|
| 61,3 g Triethylamin·2,6 HF<br>50,2 g Acetylchlorid | 0,40 Mol<br>0,64 Mol |

Aufbau und Durchführung:

In einem 250 ml Dreihalskolben mit Rückflusskühler, Thermofühler und Tropftrichter wurde der Triethylaminkomplex vorgelegt. Der Rückflusskühler wurde über einen Kryomaten mit +15 °C kalter Sole gespeist. Bei Raumtemperatur wurde langsam und unter starkem Rühren Acetylchlorid in die ölige, hellbraune Lösung getropft. Die Reaktion war leicht exotherm und die Gasentwicklung erfolgte spontan. Nach Beendigung der Zugabe wurde die Lösung noch 1 h auf 80 °C erhitzt, um die Reaktion zu vervollständigen und evtl. gelöstes Acetylfluorid auszutreiben.

Auswertung:

Zu Beginn der Zugabe von Acetylchlorid betrug die Abgaszusammensetzung 10,98 % HCl, 80,97 % Acetylfluorid, 6,59 % nicht umgesetztes Acetylchlorid und 1,46 % Essigsäure (letztere vermutlich gebildet durch in Probenahmezylinder vorhandener Feuchtigkeit; Angabe jeweils in GC %). Bei Beendigung des Zutropfens betrug die Abgaszusammensetzung 9,57 % HCl, 27,53 % Acetylfluorid, 58,01 % nicht umgesetztes Acetylchlorid und 4,89 % Essigsäure.

**Beispiel 12:**

Herstellung von Acetylfluorid aus Acetylchlorid mit Tributylamin·4,0 HF als Katalysator.

| Ansatz: | |
|---|---|
| 79,6 g Tributylamin·4,0 HF | 0,30 Mol |
| 70,7 g Acetylchlorid | 0,90 Mol |

Aufbau und Durchführung:

In einem 250 ml Dreihalskolben mit Rückflusskühler (und Blasenzähler), Thermofühler und Tropftrichter wurde der Tributylaminkomplex vorgelegt. Der Rückflusskühler wurde über einen Kryomaten mit -15 °C kalter Sole gespeist. Bei Raumtemperatur wurde langsam und unter starkem Rühren Acetylchlorid in die ölige, hellbraune Lösung getropft. Die Reaktion war leicht exotherm und die Gasentwicklung erfolgte spontan. Nach Beendigung der Zugabe wurde die Lösung noch 1 h auf 80 °C erhitzt, um die Reaktion zu vervollständigen und evtl. gelöstes Acetylfluorid auszutreiben.

Auswertung:

Zu Beginn der Zugabe von Acetylchlorid betrug die Abgaszusammensetzung 50,63 % HCl, 37,80 % Acetylfluorid, 8,15 % nicht umgesetztes Acetylchlorid und 3,43 % Essigsäure (letztere gebildet durch vorhandene Feuchtigkeit im Probenahmezylinder; Angaben jeweils in GC %). Bei Beendigung des Zutropfens betrug die Abgaszusammensetzung 31,57 % HCl, 45,96 % Acetylfluorid, 19,98 % nicht umgesetztes Acetylchlorid und 2,50 % Essigsäure.

Die Beispiele 11 und 12 belegen, dass sich Acetylfluorid auch mit Addukten mit höherem HF-Gehalt herstellen lassen.

Die Beispiele 13 bis 19 erläutern die Regenerierung ("Recycling") des durch HCl kontaminierten HF-Adduktes.

**Beispiel 13:**

Recycling von $NBu_3$·1,7 HCl zu $NBu_3$·Y HF unter Einsatz von wenig HF

$$\text{Tributylamin}\cdot 1{,}7\ \text{HCl} + \text{HF} \xrightarrow{100\ °\text{C}} \text{Tributylamin}\cdot y\text{HF}\cdot z\text{HCl}$$

Ansatz:

| Stoff | Molmasse | Masse in g | Mol |
|---|---|---|---|
| Tributylamin·1,7 HCl | 247,34 | 82,17 | 0,33 |
| HF | 20,01 | 50 | 2,5 |

In einem Laborautoklav wurde Tributylamin·1,7 HCl vorgelegt. Nach dem Verschließen wurde 50 g HF zugegeben und ca. 3 h bei 100 °C Reaktorinnentemperatur gekocht. Der Autoklav wurde dann auf ca. 60 °C Reaktorinnentemperatur abgekühlt und die Gasphase bis zum Erreichen von Atmosphärendruck im Autoklaven in eine Waschflasche mit Wasser geleitet.

Bestimmt nach der Chlorid- und Fluoridanalyse der Waschflasche hatte der im Autoklaven verbliebene Katalysator nun eine Zusammensetzung von Tributylamin·0,62 HCl·7,3 HF.

Das Beispiel zeigt, dass selbst vollständig unter Bildung von Hydrochlorid verbrauchtes HF-Addukt regeneriert werden kann. Das regenerierte Addukt konnte wieder in Fluorierungsreaktionen eingesetzt werden.

Das folgende Beispiel zeigt, dass der HCl-Gehalt dieses Produktes noch weiter abgesenkt werden kann.

**<u>Beispiel 14:</u>**

Weitere Verringerung des HCl-Gehaltes in $NBu_3$·0,62 HCl·7,3 HF zu $NBu_3$·Y HF unter Einsatz eines Überschusses an HF

$$\text{Tributylamin}\cdot 0{,}62\ \text{HCl}\cdot 7{,}3\ \text{HF} \xrightarrow{100\ ^\circ\text{C}} \text{Tributylamin}\cdot \text{Y HF} + \text{Z HCl}$$

<u>Ansatz:</u>

| Stoff | Molmasse | Masse in g | Mol |
|---|---|---|---|
| Tributylamin·0,62 HCl·7,3 HF | 354,3 | 116,83 | 0,33 |
| HF | 20,01 | 107 | 5,35 |

Zur im Autoklaven verbliebenen Mischung aus Beispiel 13 von Tributylamin·0,62 HCl·7,3 HF wurden erneut nun 107 g HF gegeben und bei ca. 100 °C nun über Nacht gekocht. Der Autoklav wurde dann auf ca. 60 °C Reaktorinnentemperatur abgekühlt und die Gasphase bis zum Erreichen von Atmosphärendruck in eine Waschflasche mit Wasser geleitet. Berechnet anhand der Chlorid- und Fluoridanalyse der Waschflasche hatte der im Autoklaven verbliebene Katalysator nun eine Zusammensetzung von Tributylamin·0,005 HCl·4,89 HF. Diese Zusammensetzung wurden durch Direktanalyse des verbliebenen Rückstandes im Autoklaven bestätigt. Die HCl war somit nahezu vollständig ausgetrieben worden. Zersetzungsprodukte des Amins wurden nicht gefunden.

Die erhaltene Zusammensetzung war hervorragend brauchbar als Fluorierungsreagenz und Fluorierungskatalysator.

**<u>Beispiel 15:</u>**

Recycling von $NEt_3$·1,0 HCl zu $NEt_3$·Y HF unter Einsatz eines Überschusses an HF

$$\text{Triethylamin}\cdot \text{HCl} + \text{HF} \xrightarrow{100\ ^\circ\text{C}} \text{Triethylamin}\cdot \text{Y HF} + \text{Z HCl}$$

Ansatz:

| Stoff | Molmasse | Masse in g | Mol |
|---|---|---|---|
| Tributylamin·HCl | 137,65 | 37,93 | 0,28 |
| HF | 20,01 | 107,7 | 5,38 |

Durchführung:

In einem Laborautoklav wurde Triethylamin Hydrochlorid vorgelegt und verschlossen. Danach wurde HF zu gegeben und über Nacht bei 100 °C Reaktorinnentemperatur gekocht. Anschließend wurde bei 100 °C Reaktortemperatur die Gasphase bis zu Atmosphärendruck in eine mit Wasser gefüllte Waschflasche abgelassen. Berechnet anhand der Chlorid- und Fluoridanalyse der Waschflasche hatte der im Autoklaven verbliebene Katalysator nun eine Zusammensetzung von Triethylamin·0,09 HCl·5,35 HF. Diese Zusammensetzung wurde durch Direktanalyse des verbliebenen Rückstandes im Autoklaven bestätigt. Die HCl war somit nahezu vollständig ausgetrieben worden. Zersetzungsprodukte des Amins wurde nicht gefunden.

**Beispiel 16:**

Einstellung des Verhältnisses von Amin zu HF auf 2,8 bei der Katalysatormischung aus Beispiel 15

Durchführung:

Die in Versuch 15 erhaltene Mischung Triethylamin·0,09 HCl·5,35 HF wurde in eine PFA-Flasche mit Fritte gefüllt und mit trockenem Stickstoff die überschüssige HF ausgetrieben. Nachdem das Gewicht der Flasche 30 min konstant geblieben ist, wurde noch 10 min ein Vakuum von $10^{-3}$ mbar angelegt um wirklich alle Restmengen HF zu entfernen. Der so erhaltene Katalysator hatte nach Chlorid-, Fluorid- und Aminanalyse nun eine Zusammensetzung von Triethylamin·2,8 HF. HCl wurde nicht mehr gefunden. Die Recycelbarkeit des Katalysators wurde hiermit bewiesen. Durch die vorgenommene Einstellung des Amin-HF-Verhältnisses werden die nucleophilen Eigenschaften des Adduktes wiederhergestellt. Es eignet sich jetzt hervorragend als Reagenz zur Herstellung von $SO_2F_2$ aus $SO_2Cl_2$.

**Beispiel 17 und 18:**

Variation der Zeitdauer der Regenerierung

$$\text{Triethylamin}\cdot\text{HCl} + \text{HF} \xrightarrow[1\,\frac{1}{4}\,\text{h}]{100\,^{\circ}\text{C}} \text{Triethylamin}\cdot\text{HF} + \text{HCl}$$

**Ansatz:**

| Stoff | Molmasse | Masse in g | Mol |
|---|---|---|---|
| Tributylamin·HCl | 137,65 | 10,00 | 0,07 |
| HF | 20,01 | 16,5 | 0,82 |

Durchführung:

In einem Laborautoklav wurde Triethylamin-Hydrochlorid vorgelegt und verschlossen. Danach wurde HF zu gegeben und 1 % Stunden bei ca. 100 °C Reaktortemperatur gekocht (Autoklav wurde 15 min. vorgeheizt). Nach dieser Zeit wurde die Gasphase innerhalb von 15 min., während der Autoklav im Ölbad stand, abgelassen und analysiert (Probe 1). Der Reaktorinhalt (18,07 g) wurde in eine PFA-Flasche gegeben und 5 min. mit Stickstoff gespült (18,02 g), aus dieser Lösung wurden 1,54 g entnommen und auf 1 l mit dest. Wasser aufgefüllt und nasschemisch analysiert (Probe 2).

| | Cl [g/l] | F [g/l] | TEA [g/l] |
|---|---|---|---|
| **Probe 1** | 1,80 | 8,49 | 0,07 |
| **Probe 2** | 0,02 | 0,80 | 0,74 |

TEA = Triethylamin

Aus den Analysendaten ergibt sich nun eine Katalysatorzusammensetzung von: **$NEt_3 \cdot 5{,}75\ HF \cdot 0{,}08\ HCl$**

**Beispiel 19:**

$$\text{Triethylamin}\cdot\text{HCl} + \text{HF} \xrightarrow{100\ ^\circ\text{C}} \text{Triethylamin}\cdot\text{HF} + \text{HCl}$$

Ansatz:

| Stoff | Molmasse | Masse in g | Mol |
|---|---|---|---|
| Tributylamin·HCl | 137,65 | 10,44 | 0,08 |
| HF | 20,01 | 21,3 | 1,06 |

Durchführung:

In einem Laborautoklav wurde Triethylamin-Hydrochlorid vorgelegt und verschlossen. Danach wurde HF zu gegeben und 30 min. bei ca. 100 °C Reaktortemperatur gekocht (Autoklav wurde 15 min. vorgeheizt). Nach dieser Zeit wurde der Autoklav aus dem Ölbad genommen und die Gasphase innerhalb von 15 min. abgelassen und analysiert (Probe 1). Der Reaktorinhalt (20,96 g) wurde in eine PFA-Flasche gegeben und 5 min. mit Stickstoff gespült(20,64 g), aus dieser Lösung wurden 1,1 g entnommen und auf 1 l mit dest. Wasser aufgefüllt und ionenchromatografisch analysiert (Probe 2).

| | Cl [g/l] | F [g/l] | TEA [g/l] |
|---|---|---|---|
| **Probe 1** | 2,66 | 1,89 | <0,10 |
| **Probe 2** | 0,02 | 0,67 | 0,42 |

Aus den Analysendaten ergibt sich eine Katalysatorzusammensetzung von : **Triethytamin·8,48 HF·0,14 HCl.**

## Patentansprüche

1. Verfahren zur Herstellung von

   a) Sulfurylchloridfluorid oder Carbonsäurefluoriden der allgemeinen Formel (I),

   RC(O)F

   worin R bedeutet: C1-C7-Alkyl; durch mindestens 1 Chloratom und/oder durch mindestens 1 Fluoratom substituiertes C1-C7-Alkyl, durch Kontaktieren von Säurechloriden und Fluorwasserstoff-Addukten von Ammoniumhydrofluorid oder Hydrofluoriden organischer Stickstoffbasen ohne Zusatz einer HF-bindenden Base oder eines HF-bindenden Lösemittels,

      a) wobei das Fluorwasserstoff-Addukt als Fluorierungsreagenz dient und nicht über die Stufe des Ammoniumhydrofluorids oder des Hydrofluorids der organischen Stickstoffbase hinaus dehydrofluoriert wird, oder

      b) wobei man mindestens 1 Mol HF pro Mol auszutauschenden Chloratom einsetzt und die Fluorwasserstoff-Addukte als Katalysator der Umsetzung der Säurechloride mit HF dienen, oder

      c) wobei die Fluorwasserstoff-Addukte regeneriert werden, um enthaltene HCl zu entfernen,

   oder

   b) zur Herstellung von Sulfurylfluorid
   durch Kontaktieren von Sulfurylchloriden und Fluorwasserstoff-Addukten von Ammoniumhydrofluorid oder Hydrofluoriden organischer Stickstoffbasen ohne Zusatz einer HF-bindenden Base oder eines HF-bindenden Lösemittels,

      a) wobei man mindestens 1 Mol HF pro Mol auszutauschenden Chloratom einsetzt und die Fluorwasserstoff-Addukte als Katalysator der Umsetzung der Säurechloride mit HF dienen, oder
      b) wobei die Fluorwasserstoff-Addukte regeneriert werden, um enthaltene HCl zu entfernen.

2. Verfahren nach Anspruch 1, **dadurch gekennzeichnet, dass** es kontinuierlich durchgeführt wird.

3. Verfahren nach Anspruch 1, **dadurch gekennzeichnet, dass** man Sulfurylfluorid aus Sulfurylchlorid oder einem Gemisch von Schwefeldioxid und Chlor herstellt, wobei das Molverhältnis von Amin oder Ammoniak und HF im Reaktionsgemisch oberhalb von 1:3 gehalten wird.

4. Verfahren nach Anspruch 1 zur Herstellung von Sulfurylfluorid unter Regeneration des HF-Adduktes, wobei man in einer ersten Stufe Sulfurylchlorid und HF miteinander umsetzt in Anwesenheit des zu regenerierenden HF-Adduktes, wobei Sulfurylchloridfluorid und regeneriertes HF-Addukt entsteht, und man in einer zweiten Stufe das gebildete Sulfurylchloridfluorid zu Sulfurylfluorid umsetzt, in Anwesenheit von HF-Addukt, mit der Maßgabe, dass in der zweiten Stufe das Mol-Verhältnis von Amin oder Ammoniak zu HF in der Reaktionsmischung oberhalb von 1:3 gehalten wird.

5. Verfahren nach Anspruch 8, **dadurch gekennzeichnet, dass** R für C1-C3-Alkyl; durch mindestens 1 Chlor-Atom und/oder durch mindestens 1 Fluor-Atom substituiertes C1-C3-Alkyl steht.

6. Verfahren nach Anspruch 5, **dadurch gekennzeichnet, dass** R für $CH_3$, $CF_3$, $CF_2Cl$, $CFCl_2$, $CCl_3$, $CHF_2$, $C_2F_5$, $C_3F_7$ steht.

7. Verfahren nach Anspruch 1, **dadurch gekennzeichnet, dass** man das HF-Addukt eines primären, sekundären oder tertiären aliphatischen Aminhydrofluorids mit bis zu 15 C-Atomen oder eines primären, sekundären oder tertiären Aminhydrofluorids mit mindestens einem aromatischen Rest einsetzt.

8. Verfahren nach Anspruch 7, **dadurch gekennzeichnet, dass** man das HF-Addukt eines Hydrofluorids eines sekundären oder tertiären aliphatischen Amins mit insgesamt bis zu 15 C-Atomen oder eines sekundären oder tertiären Amins mit einer Phenylgruppe einsetzt.

9. Verfahren nach Anspruch 8, **dadurch gekennzeichnet, dass** man das HF-Addukt von Triethylaminhydrofluorid, Tri-n-propylaminhydrofluorid, Tri-iso-propylaminhydrofluorid, Tri-n-butylaminhydrofluorid, Pyridinhydrofluorid, Piperidinhydrofluorid oder N,N-Dimethylaminhydrofluorid einsetzt.

10. Verfahren nach Anspruch 1, **dadurch gekennzeichnet, dass** man bei einer Temperatur von Umgebungstemperatur bis 150 °C arbeitet.

11. Verfahren nach Anspruch 1, **dadurch gekennzeichnet, dass** man eine Säure zusetzt.

12. Verfahren nach Anspruch 11, **dadurch gekennzeichnet, dass** man eine Halogencarbonsäure zusetzt, vorzugsweise Trifluoressigsäure.